(19) Europäisches Patentamt
European Patent Office
Office européen des brevets

(11) Publication number: **0 381 057 A2**

(12) # EUROPEAN PATENT APPLICATION

(21) Application number: **90101498.5**

(51) Int. Cl.[5]: **A61K 7/42**

(22) Date of filing: **25.01.90**

(30) Priority: **28.01.89 JP 18076/89**

(43) Date of publication of application:
**08.08.90 Bulletin 90/32**

(84) Designated Contracting States:
**DE FR GB IT**

(71) Applicant: **Sansho Seiyaku Co., Ltd.**
**26-7, Oike 2-chome**
**Onojo-shi Fukuoka-ken(JP)**

(72) Inventor: **Okazaki, Kiyotaka**
**123-18, Yoshimatsu**
**Dazaifu-shi, Fukuoka-ken(JP)**

(74) Representative: **Patentanwälte Deufel- Schön- Hertel- Lewald- Otto**
**Isartorplatz 6**
**D-8000 München 2(DE)**

(54) **Chemical composition for external application.**

(57) Disclosed is a chemical composition for external application, which comprises a kojic acid or kojic acid derivative and an unsaturated linear fatty acid having from 4 to 26 carbon atoms and having from 1 to 6 unsaturated bonds where the position(s) of the unsaturated bond(s) is (are) any desired position(s) in the alkyl chain of the acid except the terminal opposite to the carboxyl group thereof or a derivative thereof as active ingredients. By the synergistic effect of the two active ingredients, the composition is effective for noticeably preventing formation of melanin to cause freckles and spots in the skin and for noticeably whitening the skin.

EP 0 381 057 A2

# CHEMICAL COMPOSITION FOR EXTERNAL APPLICATION

## BACKGROUND OF THE INVENTION

### Field of the Invention

The present invention relates to a chemical composition of external application, which comprises a kojic acid or a kojic acid derivative and an unsaturated linear fatty acid or a derivative as active ingredients and which is effective for preventing chromatosis that is caused by formation of melanin, such as freckles or spots, and has an excellent skin-whitening effect.

### Prior Art

Hitherto, kojic acid and kojic acid derivatives have been known as substances that inhibit the formation of melanin.

Various skin-whitening cosmetic compositions which contain the substances as an active ingredient have been disclosed (for example, JP-B-56-18569, and JP-A-56-7710, 56-7776 and 56-79616). (The terms "JP-B" and "JP-A" as used herein mean an "examined Japanese patent publication" and an "unexamined published Japanese patent application", respectively.)

In addition, flavonol compounds are also known to have the same activity. For instance, JP-A-55-92305 and 57-14517 disclose cosmetic compositions and skin liniments containing quercetin as an active ingredient. JP-B-58-34477 discloses compositions containing a quercetin fatty acid ester as an active ingredient; and JP-A-55-111410 and 55-35506 disclose compositions containing myricetin, fisetin or rhamnetin as an active ingredient. JP-A-55-111410 and 55-143908 disclose composition containing a 3-hydroxychromone compound as an active ingredient.

The present inventors investigated to find a new substance having a safe and effective activity for inhibiting formation of melanin and broadly studied the effect of various related substances with respect to the endermic applicability thereof and, as a result, previously found that unsaturated fatty acids of 10-pentadecenic acid and 9-hexadecenic acid are effective for that purpose. Accordingly, they invented a chemical composition for external application which contains the compound as an active ingredient (Japanese Patent Application No. 63-213255). In addition, they further found that the unsaturated linear fatty acids other than the said acids, which have from 4 to 17 carbon atoms and from 1 to 4 unsaturated bonds and in which the position(s) of the unsaturated bonds(s) is (are) in the alkyl chain of the acid except the terminal opposite to the carboxyl group thereof, as well as derivatives thereof have an excellent effect of inhibiting formation of melanin. Accordingly, they also invented another chemical composition for external application which contains the substance as an active ingredient (Japanese Patent Application No. 63-284128).

## SUMMARY OF THE INVENTION

The object of the present invention is to provide a chemical composition for external application, which has a more outstanding effect of inhibiting formation than the above-mentioned unsaturated linear fatty acids and derivatives.

The present inventor further investigated various unsaturated linear fatty acids having an activity of inhibiting formation of melanin and, as a result, has found that a particular combination comprising an unsaturated linear fatty acid having from 4 to 26 carbon atoms and from 1 to 6 unsaturated bonds or a derivative thereof and a kojic acid or kojic acid derivative which has been known as a melanin formation-inhibiting substance displays an excellent effect of inhibiting formation of melanin because of the synergistic effect of the combined components. Accordingly, he has hereby achieved the present invention on the basis of such findings.

Specifically, the present invention provides a chemical composition for external application, which comprises a kojic acid or kojic acid derivative and an unsaturated linear fatty acid having from 4 to 26

carbon atoms and having from 1 to 6 unsaturated bonds where the position(s) of the unsaturated bond(s) is-(are) any desired position(s) in the alkyl chain of the acid accept the terminal opposite to the carboxyl group thereof or a derivative thereof as active ingredients.

## DETAILED EXPLANATION OF THE INVENTION

The kojic acid or kojic acid derivatives are 5-oxy-2-hydroxymethyl-γ-pyrone and derivatives thereof, which are produced from various hydrocarbon compounds by the action of kojic acid-producing strains belonging to the genus Aspergillus or Penicillium.

The kojic acid derivatives includemono fatty acid esters of kojic acid such as kojic acid monopalmitate, kojic acid monobutyrate, kojic acid monocaprylate and kojic acid monostearate (refer to JP-A-56-77272), difatty acid esters of kojic acid such as kojic acid dipalmitate, kojic acid dioleate, kojic acid dibutyrate and kojic acid distearate (refer to JP-A-56-7776), as well as kojic acid monocinnamoate and kojic acid monobenzoate (refer to JP-A-59-33207)

The other active ingredient which constitutes the chemical composition of the present invention is an unsaturated linear fatty acid having from 4 to 26 carbon atoms and having from 1 to 6 unsaturated bonds, and the position(s) of the unsaturated bond(s) is (are) any desired position(s) in the alkyl chain of the acid except the terminal opposite to the carboxyl group thereof. This includes, for example, 2-butenoic acid ($C_{4\ (number\ of\ carbon\ atoms)}$: 1 (number of unsaturated bond)Δ2(position of unsaturated bond in the alkyl chain as counted from the carboxyl group-bonded carbon atom of the alkyl chain) - the same shall apply hereunder), 6-octenoic acid ($C_8$:1Δ6), 6-decenoic acid ($C_{10}$:1Δ6), 7- undecylenoic acid ($C_{11}$:1Δ7), 9-dodecenoic acid ($C_{12}$:1Δ9), 6,9-dodeoadienoic acid ($C_{12}$:2Δ6-9), 10-tridecenoic acid ($C_{13}$:1Δ10), 10-pentadecenoic acid ($C_{15}$:1Δ10), 7,10-tridecadienoic acid ($C_{13}$:2Δ7-10), 6,9-tetradecadienoic acid ($C_{14}$:2Δ6-9), 7,10-pentadecadienoic acid ($C_{15}$:2Δ7-10), 10-heptadecenoic acid ($C_{17}$:1Δ10), 6,9-hexadecadienoic acid ($C_{16}$:2Δ6-9), 6,9,12-hexadecatrienoic acid ($C_{16}$:3Δ6-9-12), myristoleic acid ($C_{14}$:1Δ9), palmitoleic acid ($C_{16}$:1Δ9) oleic acid ($C_{18}$:1Δ9), linoleic acid ($C_{18}$:2Δ9-12), linolenic acid ($C_{18}$:3Δ9-12-15), homo-γ-linolenic acid ($C_{20}$:3Δ8-11-14), arachidonic acid ($C_{20}$:4Δ5, 8-11-14).

Derivatives of the said unsaturated linear fatty acids which may be employed in the present invention include the following compounds:

(1) Monoglycerides of the following formula (I) or (II):

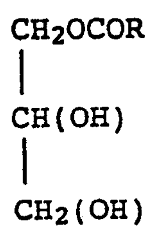
( I )
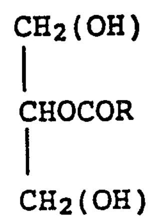
( II )

wherein R represents an aliphatic residue of the above-mentioned unsaturated linear fatty acid.

(2) Diglycerides of the following formula (III) or (IV):

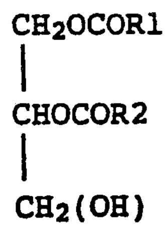
( III )
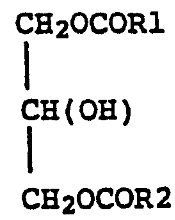
( IV )

wherein R1 and R2 each represents a chain-type organic group, and at least one of them means an aliphatic residue of the above-mentioned unsaturated linear fatty acid.

(3) Triglycerides of the following formula (V):

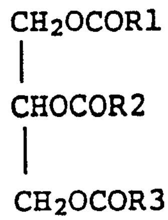

(V)

wherein R1, R2 and R3 each represent a chain-type organic group, and at least one of them means an aliphatic residue of the above-mentioned unsaturated linear fatty acid.

(4) Metal salts of the following formula (VI):

(RCOO)pM (VI)

wherein R represents an aliphatic residue of the above-mentioned unsaturated linear fatty acid; M represents a metal atom; and P represents the valency of M.

(5) Esters of the following formula (VII)

RCOOR′ (VII)

wherein R represents an aliphatic residue of the above-mentioned unsaturated linear fatty acid; and R′ represents a monohydric or polyhydric alcohol residue, a polyoxyethylene residue, a sorbitan residue or a saccharide residue.

(6) Phospholipids of the following formula (VIII):

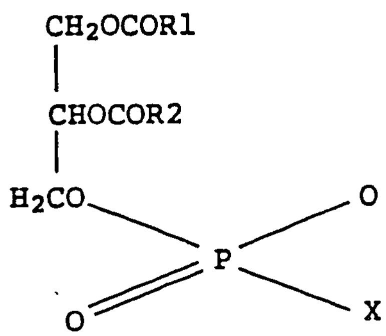

(VIII)

wherein R1, and R2 each represent a chain-type organic group, and at least one of them means an aliphatic residue of the above-mentioned unsaturated linear fatty acid; and X represents a choline, ethanolamine, serine, glycerol, phospholipid or inositol residue.

(7) Phosphatidic acids of the following formula (IX):

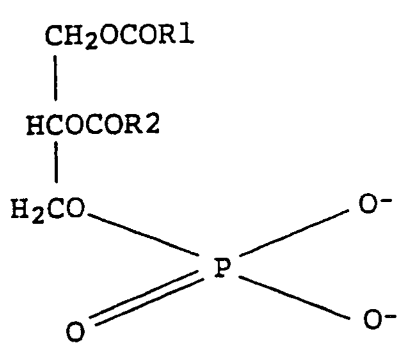

(IX)

wherein R1 and R2 each represent a chain-type organic group, and at least one of them means an aliphatic residue of the above-mentioned unsaturated linear fatty acid.

(8) Sterol esters of the following formula (X):

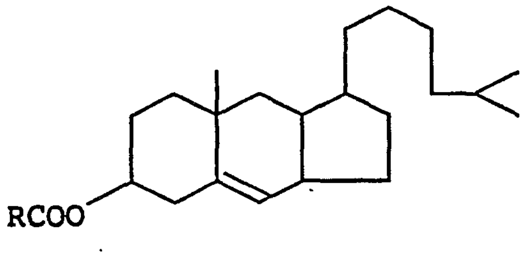

(X)

wherein R represents an aliphatic residue of the above-mentioned unsaturated linear fatty acid.

(9) Sphingolipids of the following formula (X I ):

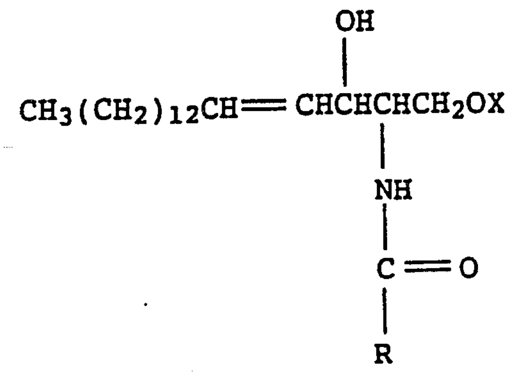

(X I )

wherein R represents an aliphatic residue of the above-mentioned unsaturated linear fatty acid; and X represents a saccharide, phosphoric acid or amine salt residue.

(10) Primary amides of the following formula (XII):

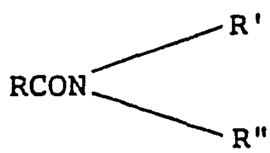

(X II)

wherein R represents an aliphatic residue of the above-mentioned unsaturated linear fatty acid; and R′ and R″ each represent a hydrogen atom or an organic group.

(11) Secondary amides of the following formula (XIII):

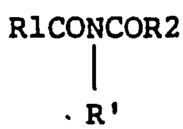

(X III)

where R1 and R2 each represent a chain-type organic group, and at least one of them means an aliphatic residue of the above-mentioned unsaturated linear fatty acid; and R′ represents a hydrogen atom or an organic group.

(12) Tertiary amides of the following formula (XIV):

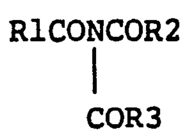

(X IV)

wherein R1, R2 and R3 each represent a chain-type organic group, and at least one of them means an

aliphatic residue of the above-mentioned unsaturated linear fatty acid.

(13) Dibasic acids of the following formula (XV) and salts thereof:

HOOCRCOOH (XV)

wherein R represents an aliphatic residue of the above-mentioned unsaturated linear fatty acid.

As derivatives of the above-mentioned compounds, there are mentioned, for example, mono-, di-, and tri-glycerides such as 1-mono-6-octenoin, 1-mono-9-dodecenoin,1-monomyristolein, 1-mono-10-pentadecenoin, 1-monopalmitolein, 1-mono-10-heptadecenoin, 2-mono-3-hexenoin, 2-mono-7-undecenoin, 2-monopentadecenoin, 2-monopalmitolein, 2-mono-10-heptadecenoin, 1,2-di-9- dodecenoin, 1,2-dimyristolein, 1,2-di-10-pentadecenoin, 1,2-dipalmitolein, 1,3-dimyristolein, 1,3-di-10-pentadecenoin, 1,3-dipalmitolein, 1,3-di-10-heptadecenoin, trimyristolein, tri-10-pentadecenoin, tripalmitolein and tri-10-heptadecenoin; esters such as methyl 9-myristoleate, methyl 10-pentadecenoate, methyl palmitoleate and methyl 10-heptadecenoate; phosphatidic acid salts such as disodium dipalmitoleylphosphatidate disodium and di-10-pentadecenylphosphatidate; sphingolipids such as N-palmitoley-D-sphingomyelin and N-10 pentadecenyl-D-sphingomyelin; amides such as palmitoleic acid amide, dipalmitoleic acid amide and tripalmitoleic acid amide; dibasic acids such as 9-hexadecenedicarboxylic acid; and sterol esters such as palmitoleyl cholesterol and myristoleyl cholesterol.

The chemical composition for external application of the present invention is intended to include cosmetic compositions such as cream, facial wash or pack, as well as quasi drugs for external application such as emulsion, lotion or liniment.

In accordance with the present invention, the chemical composition for external application contains the above-mentioned kojic and or kojic acid derivative and the above-mentioned unsaturated linear fatty acid or derivative thereof as the active ingredient in an amount of from 0.001 to 5 % by weight, preferably from 0.01 to 1 % by weight, for the cosmetic composition or in an amount of from 0.01 to 10 % by weight, preferably from 0.01 to 2 % by weight, for the quasi drugs for external application.

Where the chemical composition of the present invention is prepared, any conventional base and aid which are generally employed in preparation of ordinary cosmetic composition or quasi drugs for external application may be employed along with the above-mentioned active ingredients in a conventional manner.

Preferably, the chemical composition of the present invention contains various ultraviolet absorbents such as benzophenone compounds, cinnamic acid compounds, salicylic acid compounds, benzoic acid compounds, ethyl urocanate, benzotriazole or vegetable-derived substances; as well as various antioxidants such as vitamin E, BHA, BHT, erisorbic acid, phytic acid, nordihydroquaiaretic acid or propyl gallate.

Next, the present invention will be explained in more detail by way of the following examples, which, however, do not restrict the scope of the present invention. "%" in the examples is by weight.

EXAMPLE 1:

| Cream: | |
|---|---|
| 9-Tetradecenoic Acid | 0.05 % |
| Kojic Acid | 0.7 % |
| Polyoxyethylene Stearyl Ether | 2.0 % |
| Polyoxyethylene Cetyl Ether | 2.0 % |
| Bee Wax | 6.0 % |
| Cetanol | 6.0 % |
| Isopropyl Palmitate | 10.0 % |
| Liquid Paraffin | 30.0 % |
| Liethylene Glycol Monostearate | 1.0 % |
| Methyl Parahydroxybenzoate | 0.2 % |
| Pure Water to make | 100.0 % |

EXAMPLE 2:

| Emulsion: | |
|---|---|
| Kojic Acid | 1.0 % |
| 7,10-Pentadecadienoic Acid | 0.1 % |
| Self-emulsifying Glycerol Monostearate | 0.5 % |
| Polyoxyethylene Cetyl Ether | 2.0 % |
| MC Stearic Acid | 2.0 % |
| Cetanol | 1.5 % |
| Isopropyl Mydistate | 10.0 % |
| Methyl Parahydroxybenzoate | 0.2 % |
| Perfume | Trace |
| Pure Water to make | 100.0% |

EXAMPLE 3

| Pack: | |
|---|---|
| Kojic Acid | 0.8 % |
| 10-Heptadecenoic Acid | 1.0 % |
| Ethanol | 6.0 % |
| Methyl Parahydroxybenzoate | 0.08 % |
| Carboxy Vinyl Polymer | 1.0 % |
| Calcium Carbonate | 0.6 % |
| Titanium Dioxide | 0.02 % |
| Perfume | Trace |
| Pure Water to make | 100.0 % |

EXAMPLE 4

| Lotion: | |
|---|---|
| Kojic Acid | 0.5 % |
| Liposome Liquid of 6,9-Hexadecadienoic Acid (containing 1 % of 9-hexadecenoic acid) | 0.5 % |
| Parahydroxybenzoic Acid | 0.08 % |
| Citric Acid | 0.3 % |
| Perfume | Trace |
| Pure Water to make | 100.0 % |

The melanin formation-inhibiting activity of the unsaturated linear fatty acids and derivatives thereof which are the active ingredients of the composition of the present invention will be substantiated by the following experimental example.

EXPERIMENTAL EXAMPLE:

This illustrates the test of formation of melanin by B16 cells.

Mouse melanoma-derived B16 cells (4 x $10^4$ cells) were suspended in an Eagle MEM medium containing 10 ml of fetal calf serum (FCS) and incubated in a 25 cc-Roux incubator flask at 37° C in the presence of 5 % $CO_2$ . On the first day and the third day, the medium was exchanged by a medium containing 80 µM/liter and the incubation was carried out for 5 days. The cells were washed with a

phosphate buffer (pH 7.20 containing 0.8 W/V of sodium chloride and then separated by the use of trypsine and EDTA-containing solution. Thereafter the cells were recovered by filtration. The cells as recovered on the filter were measured with a densitometer and the reflection density (blacking density) thereof was obtained.

In the test, the concentration of the kojic acid tested was 0.5, 1.0, 1.5 and 2.0 mM, and the whitened degree of each case was obtained. The results are shown in Table 1 below.

Table 1

| Whitening Test of Incubated Color Cells with Kojic Acid | | | | |
|---|---|---|---|---|
| Test Concentration (mM) | 0.5 | 1.0 | 1.5 | 2.0 |
| Whitened Degree | - | - | - | + |

In the same test, various unsaturated linear fatty acids of 6-octenoic acid, myristoleic acid, 10-pentadecenoic acid, palmitoleic acid, oleic acid, linolenic acid, homo-γ-linolenic acid or arachidonic acid were employed singly, each in a concentration of 0.001, 0.01, 0.1, or 0.5 mM, and the whitened degree of each case was also obtained. The results are shown in Table 2 below.

Table 2

| Whitening Test of Incubated Color Cells with Fatty Acid | | | | |
|---|---|---|---|---|
| Test Concentration (mM) Test Compound | 0.001 | 0.01 | 0.1 | 0.5 |
| 6-Octenoic Acid | - | - | + | + |
| Myristoleic Acid | - | - | + | + |
| 10-Pentadecenoic Acid | - | - | + | + |
| Palmitoleic Acid | - | - | + | + |
| Oleic Acid | - | - | + | + |
| Linolic Acid | - | - | + | + |
| Linolenic Acid | - | - | + | + |
| Homo-γ-linolenic Acid | - | - | + | + |
| Arachidonic Acid | - | - | + | + |

In the same test, kojic acid was combined with any one of myristoleic acid, 10-pentadecenoic acid, palmitoleic acid, oleic acid, linolic acid, linolenic acid, homo-γ-linolenic acid or arachidonic acid whereupon the concentration of the former was 0, 0.5, 1.0, 1.5, or 2.0 mM and that of the latter was 0, 0.001, 0.01, 0.1 or 0.5mM, in accordance with the present invention, and the whitened degree of each case was also obtained. The results are shown in Tables 3-1 to 3-8 below.

Table 3-1

| Concentration of Kojic Acid (mM) | Concentration of Myristoleic Acid (mM) | | | | |
|---|---|---|---|---|---|
| | 0 | 0.001 | 0.01 | 0.1 | 0.5 |
| 0 | - | - | - | + | + |
| 0.5 | - | - | - | + | + |
| 1.0 | - | - | - | + | + |
| 1.5 | - | - | + | + | + |
| 2.0 | + | + | + | + | + |

Table 3-2

| Concentration of Kojic Acid (mM) | Concentration of 10-Pentadecenoic Acid (mM) | | | | |
|---|---|---|---|---|---|
| | 0 | 0.001 | 0.01 | 0.1 | 0.5 |
| 0 | - | - | - | + | + |
| 0.5 | - | - | - | + | + |
| 1.0 | - | - | + | + | + |
| 1.5 | - | + | + | + | + |
| 2.0 | + | + | + | + | + |

Table 3-3

| Concentration of Kojic Acid (mM) | Concentration of Palmitoleic Acid (mM) | | | | |
|---|---|---|---|---|---|
| | 0 | 0.001 | 0.01 | 0.1 | 0.5 |
| 0 | - | - | - | + | + |
| 0.5 | - | - | - | + | + |
| 1.0 | - | - | + | + | + |
| 1.5 | - | + | + | + | + |
| 2.0 | + | + | + | + | + |

Table 3-4

| Concentration of Kojic Acid (mM) | Concentration of Oleic Acid (mM) | | | | |
|---|---|---|---|---|---|
| | 0 | 0.001 | 0.01 | 0.1 | 0.5 |
| 0 | - | - | - | + | + |
| 0.5 | - | - | - | + | + |
| 1.0 | - | - | - | + | + |
| 1.5 | - | - | + | + | + |
| 2.0 | + | + | + | + | + |

Table 3-5

| Concentration of Kojic Acid (mM) | Concentration of Linolic Acid (mM) | | | | |
|---|---|---|---|---|---|
| | 0 | 0.001 | 0.01 | 0.1 | 0.5 |
| 0 | - | - | - | + | + |
| 0.5 | - | - | - | + | + |
| 1.0 | - | - | - | + | + |
| 1.5 | - | - | + | + | + |
| 2.0 | + | + | + | + | + |

Table 3-6

| Concentration of Kojic Acid (mM) | Concentration of Linolenic Acid (mM) | | | | |
|---|---|---|---|---|---|
| | 0 | 0.001 | 0.01 | 0.1 | 0.5 |
| 0 | - | - | - | + | + |
| 0.5 | - | - | - | + | + |
| 1.0 | - | - | - | + | + |
| 1.5 | - | - | + | + | + |
| 2.0 | + | + | + | + | + |

Table 3-7

| Concentration of Kojic Acid (mM) | Concentration of Homo-γ-linoleic Acid (mM) | | | | |
|---|---|---|---|---|---|
| | 0 | 0.001 | 0.01 | 0.1 | 0.5 |
| 0 | - | - | - | + | + |
| 0.5 | - | - | - | + | + |
| 1.0 | - | - | - | + | + |
| 1.5 | - | - | + | + | + |
| 2.0 | + | + | + | + | + |

Table 3-8

| Concentration of Kojic Acid (mM) | Concentration of Arachidonic Acid (mM) | | | | |
|---|---|---|---|---|---|
| | 0 | 0.001 | 0.01 | 0.1 | 0.5 |
| 0 | - | - | - | + | + |
| 0.5 | - | - | - | + | + |
| 1.0 | - | - | - | + | + |
| 1.5 | - | - | + | + | + |
| 2.0 | + | + | + | + | + |

In the Tables above, (+) indicates that the density was noticeably thinned down, while (-) indicates no substantial change.

As explained in detail in the above, the chemical composition for external application of the present invention is effective for noticeably preventing formation of freckles and spots in the skin and whitening the skin. It can be used safely, without causing any skin troubles such as skin irritations or eruptions. Accordingly, the present invention is extremely useful.

While the invention has been described in detail and with reference to specific embodiments thereof, it will be apparent to one skilled in the art that various changes and modifications can be made therein without departing from the spirit and scope thereof.

## Claims

1. A chemical composition for external application, which comprises a kojic or kojic acid derivative and an unsaturated linear fatty acid having from 4 to 26 carbon atoms and having from 1 to 6 unsaturated bonds where the position(s) of the unsaturated bond(s) is (are) any desired position(s) in the alkyl chain of the acid except the terminal opposite to the carboxyl group thereof or a derivative thereof as active ingredients.

2. The chemical composition for external application as claimed in claim 1, in which the kojic acid derivative is one selected from the group consisting of mono fatty acid esters of kojic acid, difatty acid esters of kojic acid, kojic acid monocinnamoate and kojic acid monobenzoate.

3. The chemical composition for external application as claimed in claim 1, in which the unsaturated linear fatty acid derivative is a monoglyceride of a formula (I) or (II):

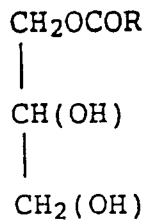

( I )

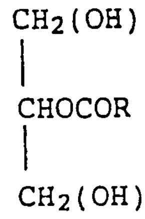

( II )

wherein R represents an aliphatic residue of an unsaturated linear fatty acid.

4. The chemical composition for external application as claimed in claim 1, in which the unsaturated linear fatty acid derivative is a diglyceride of a formula (III) or (IV):

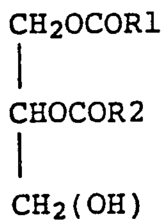

( III )

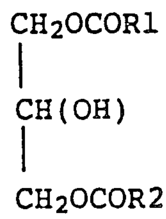

( IV )

wherein R1 and R2 each represent a chain-type organic group, and at least one of them means an aliphatic residue of an unsaturated linear fatty acid.

5. The chemical composition for external application as claimed in claim 1, in which the unsaturated linear fatty acid derivative is a triglyceride of a formula (V):

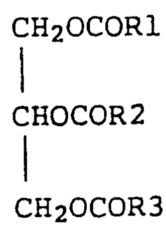

( V )

wherein R1, R2 and R3 each represent a chain-type organic group, and at least one of them means an aliphatic residue of an unsaturated linear fatty acid.

6. The chemical composition for external application as claimed in claim 1, in which the unsaturated linear fatty acid derivative is a metal salt of a formula (VI):

(RCOO)pM (VI)

wherein R represents an aliphatic residue of an unsaturated linear fatty acid; M represents a metal atom; and prepresents the valency of M.

7. The chemical composition for external application as claimed in claim 1, in which the unsaturated linear fatty acid derivative is an ester of a formula (VII)

RCOOR′ (VII)

wherein R represents an aliphatic residue of an unsaturated linear fatty acid; and R′ represents a monohydric or polyhydric alcohol residue, polyoxyethylene residue, sorbitan residue or saccharide residue.

8. The chemical composition for external application as claimed in claim 1, in which the unsaturated linear fatty acid derivative is a phospholipid of formula (VIII):

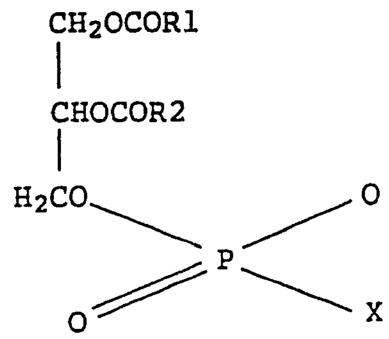

( VIII )

wherein R1 and R2 each represent a chain-type organic group, and at least one of them means an aliphatic residue of an unsaturated linear fatty acid; and X represents a choline, ethanolamine, serine, glycerol, phospholipid or inositol residue.

9. The chemical composition for external application as claimed in claim 1, in which the unsaturated linear fatty acid derivative is a phosphatidic acid of a formula (IX):

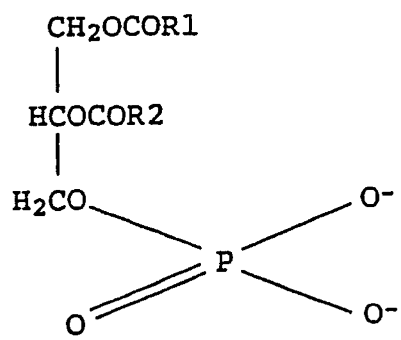

( IX ) )

wherein R1 and R2 each represent a chain-type organic group, and one of them means an aliphatic residue of unsaturated linear fatty acid.

10. The chemical composition for external application as claimed in claim 1, in which the unsaturated linear fatty acid derivative is a sterol ester of a formula (X):

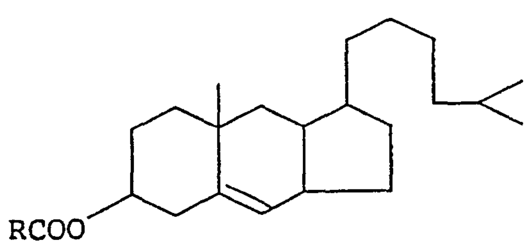

( X )

wherein R represents an aliphatic residue of an unsaturated linear fatty acid.

11. The chemical composition for external application as claimed in claim 1, in which the unsaturated linear fatty acid derivative is a sphingolipid of a formula (XI):

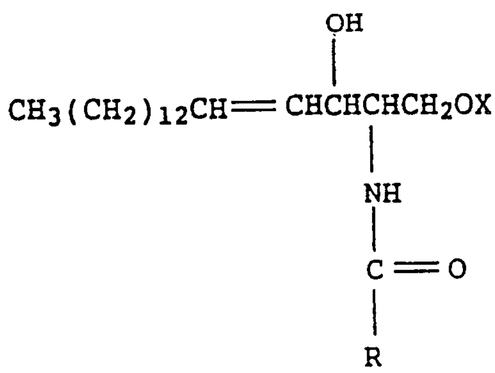

(X I)

wherein R represents an aliphatic residue of an unsaturated linear fatty acid; and X represents a saccharide, phosphoric acid or amine salt residue.

12. The chemical composition for external application as claimed in claim 1, in which the unsaturated linear fatty acid derivative is a primary amide of a formula (XII):

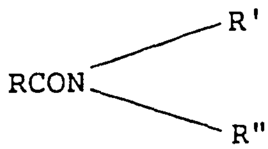

(XII)

wherein R represents an aliphatic residue of an unsaturated linear fatty acid; and R and $R''$ each represent a hydrogen atom or an organic group.

13. The chemical composition for external application as claimed in 1, in which the unsaturated linear fatty acid derivative is a secondary amide of a formula (XIII):

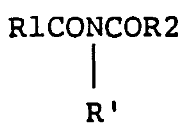

(XIII)

wherein R1 and R2 each represent a chain-type organic group, and at least one of them means an aliphatic residue of an unsaturated linear fatty acid; and $R'$ represents a hydrogen atom or an organic group.

14. The chemical composition for external application as claimed in claim 1, in which the unsaturated linear fatty acid derivative is a tertiary amide of a formula (XIV):

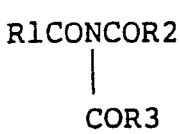

(XIV)

wherein R1, R2 and R3 each represent a chain-type organic group, and at least one of them means an aliphatic residue of an unsaturated linear fatty acid.

15. The chemical composition for external application as claimed in claim 1, in which the unsaturated linear fatty acid derivative is a dibasic acid of a formula (XV) and salts thereof:

HOOCRCOOH (XV)

wherein R represents an aliphatic residue of an unsaturated linear fatty acid, or a salt thereof.

16. The chemical composition for external application as claimed in any one of claims 1 to 15, which is in the form of a cosmetic composition of cream, face wash or pack.

17. The chemical composition for external application as claimed in any one of claims 1 to 16, in which the content of the active ingredients of the kojic acid or kojic acid derivative and the unsaturated linear fatty acid or derivative thereof is from 0.001 to 5 % by weight.

18. The chemical composition for external application as claimed in claim 17, in which the content of

the active ingredients of the kojic acid or kojic acid derivative and the unsaturated linear fatty acid or derivative thereof is from 0.01 to 1 % by weight.

19. The chemical composition for external application as claimed in any one of claims 1 to 15, which is in the form of a quasi medicament for external application of emulsion, lotion or liniment.

20. The chemical composition for external application as claimed in any one of claims 1 to 15 and 19, in which the content of the active ingredients of the kojic acid or kojic acid derivative and the unsaturated linear fatty acid or derivative thereof is from 0.01 to 10 % by weight.

21. The chemical composition for external application as claimed in claim 20, in which the content of the active ingredients of the kojic acid or kojic acid derivative and the unsaturated linear fatty acid or derivative thereof is from 0.01 to 2 % by weight.